# EUROPEAN PATENT SPECIFICATION

(11) **EP 2 164 564 B1**
(45) Date of publication and mention of the grant of the patent: **21.08.2013**
(21) Application number: 07748292.5
(22) Date of filing: 27.06.2007
(51) Int. Cl.: A61N 1/362, A61B 5/053, A61N 1/37

(54) **A DEVICE FOR COLLECTING REM SLEEP DATA**
VORRICHTUNG ZUR SAMMLUNG VON REM-SCHLAFDATEN
DISPOSITIF DESTINÉ À LA COLLECTE DE DONNÉES RELATIVES AU SOMMEIL PARADOXAL

(43) Date of publication of application: 24.03.2010
(73) Proprietor: St. Jude Medical AB, 175 84 Järfälla (SE)
(72) Inventor: BLOMQVIST, Andreas, S-187 76 Täby (SE); BROOME, Michael, S-178 38 Ekerö (SE)
(74) Representative: Sjölander, Henrik
(86) International application number: PCT/SE2007/000632
(87) International publication number: WO 2009/002238

(56) References cited:
- EP-A2- 0 450 341
- WO-A1-2005/018737
- WO-A1-2007/064682
- US-A1- 2005 043 652
- US-A1- 2005 209 512
- US-A1- 2007 055 115
- US-A1- 2007 270 706

## Description

### TECHNICAL FIELD

The present invention relates to an implantable medical device such as a pacemaker or an implantable cardioverter or any other similar device. In particular the present invention relates to a device for detecting REM sleep using an implantable medical device.

### BACKGROUND

In implantable medical devices such as pacemakers and implantable cardioverter/defibrillators a number of different sensors are provided. The sensors are i.a. used for controlling the operation of the implantable medical device. In recent years an increased memory capacity in implantable medical devices has paved the way for introduction of diagnostic abilities for trending various conditions and diseases. In some cases signals collected are even used for prediction of critical events.

In trending of various conditions as well as in prediction of events it is desired to have access to data generated under stable conditions. In other words when data is compared to historical data it is desired that the historical data is generated under conditions that are as similar as possible, thereby improving the accuracy of the trended parameter.

If data related to trending and/or prediction of different conditions are collected during conditions that differ, there is a high risk that the trend in the trended parameter is lost because the variation in the conditions is larger than the difference in the trended parameter. This in turn will possibly result in that a significant change in a trended parameter can not be detected. This is of course not desired. Also, US 20050043652 discloses an implantable medical device comprising a sleep state processor that uses information from sleep state sensors to determine REM and non-REM states.

WO 2005/018737 describes a sleep stage discrimination circuitry including a medical device coupled to sleep stage sensors, cardiac system sensors and respiratory system sensors. The cardiac sensors may be used to sense the electrical activity of the heart. The respiratory system sensors are capable of detecting conditions associated with the respiratory functions of the patient and may include a transthoracic impedance sensor. Furthermore, the medical device includes a sleep/sleep stage detector that uses information from the sleep stage sensors to determine the states of the patient's sleep including, for example, REM and non-REM states. REM sleep states may be detected by utilizing REM-modulated signals, which may be sensed by means of, for example, electroencephalogram electrodes for detecting brain activity, electrooculogram sensors for detecting eye movement, sensors for detecting muscle atonia including electromyogram sensors, strain gauge sensors, piezoelectric sensors, mechanical force sensors, or other transducers. Sleep stage information may be provided to a therapy module coupled to the sleep/sleep stage detector. The therapy module controls the delivery of sleep stage informed therapy to the patient. For example, cardiac therapy may be coordinated using sleep stage classification information to provide cardiac arrhythmia therapy during REM or other proarrhythmic sleep periods. Sleep stage classification may also be used, for example, in connection with delivery of sleep informed therapy to preclude or reduce episodes of disordered breathing while the patient is asleep.

Hence, there exists a need for a method and a device that is capable of generating an output of a trended parameter that is reliable and which can capture changes in a trended parameter with high accuracy.

### SUMMARY

It is an object of the present invention to provide a device that is capable of detecting REM sleep using signals that are captured by an implantable medical device.

It is another object of the present invention to overcome or at least reduce some of the problems associated with existing methods and devices for trending various conditions/deceases using an implantable medical device.

It is yet another object of the present invention to provide a device that is capable of generating data for trending a physiological parameter at conditions that are similar over a period of time.

These objects and others are obtained by the device as set out in the appended claims. Thus, by providing an REM sleep detection circuitry in an implantable medical device that based on sensor signals available to the implantable medical device, in particular an impedance sensor signal, can determine if a person is in REM sleep, an REM detector that is reliable and which can be used without any external input is obtained.

An impedance signal used for determining an REM sleep stage can be supplemented with other signal available in an implantable medical device in order to increase the accuracy of the determination whether an REM sleep stage exists or not. Such other signals may include, but are not limited to signals indicative of, heart rate, heart rate variability, position sensor signals, accelerometer signals, SvO2 sensor signals and blood pressure sensor signals.

The impedance can be measured between any electrode(s) present in the implantable device and the casing of the implantable device or between any electrodes of the implantable device. The measured impedance signal is then used as an input signal to an REM sleep detector. For example, the REM detector can be adapted to extract the heart rate and/ or the heart rate variability from the impedance signal provided. The IEGM signal is a good indicator of REM sleep because an increased heart rate indicates REM sleep. Also an increased variability of the heart rate is a good indicator of REM sleep.

In another embodiment an REM detector is used for determining when and/ or how trend data for use in trending should be recorded. Hence, because REM sleep is different from regular sleep if data is recorded both during an REM sleep condition and during regular sleep the data will be corrupt or at least not representing conditions that are similar. In order to combat this problem data used for trending is separated so that data collected during REM sleep is classified as one set of data whereas data collected during regular sleep is classified as another set of data. Hereby trending can be made on data that are collected during conditions that are similar and which is not affected by different sleep stages.

In yet another embodiment of the present invention, the ratio between a trended parameter during REM sleep and during regular sleep is formed to provide a measurement useful for detecting a worsened condition.

### BRIEF DESCRIPTION OF THE DRAWINGS

The present invention will now be described in more detail by way of non-limiting examples and with reference to the accompanying drawings, in which:
- Fig. 1 is a general view of an implanted medical device
- Fig. 2a is a general view illustrating the major logical components of an REM detector adapted to be integrated in an implantable medical device,
- Fig. 2b is a schematic view of a heart beat signal used for post capturing detection of REM sleep periods.
- Fig. 3 is a flow chart illustrating steps performed when detecting REM sleep, and
- Fig. 4 is a flow chart illustrating different steps performed when collecting trending data in an implantable medical device provided with an REM detector.
- Fig. 5 is a view illustrating how an implantable medical device can be made to communicate with other electronic devices located outside a patient carrying an implanted medical device

### DETAILED DESCRIPTION

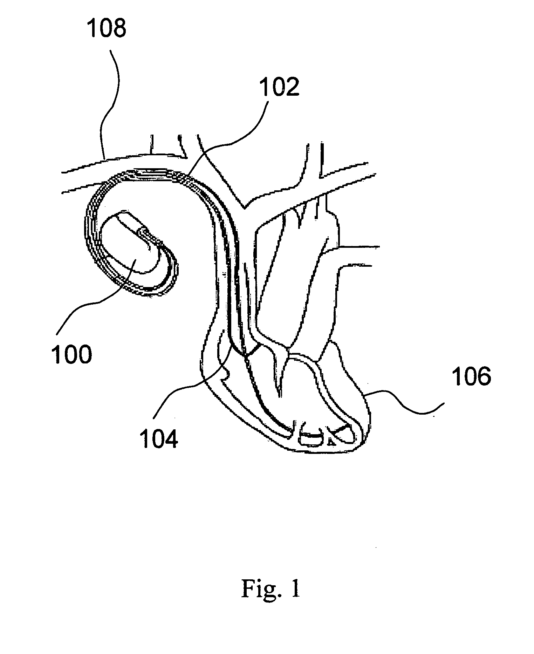

In Fig. 1, a view of a medical device 100 implanted in a patient is shown. The exemplary medical device can for example be a pacemaker implanted in a patient. The pacemaker 100 comprises a casing being hermetically sealed and biological inert. The casing is typically conductive and may then serve as an electrode. One or more pacemaker leads, whereof only two are shown in Fig. 1 namely a ventricular lead 102 and an atrial lead 104, are electrically coupled to the pacemaker 100 in a conventional manner. The leads 102 and 104 extend into the heart 106 via a vein 108 of the patient. One or more conductive electrodes for receiving electrical cardiac signals and/or for delivering electrical pacing to the heart 106 are arranged near the distal ends of the leads 102 and, 104. Impedance measurements may be performed between any of the electrodes, the casing of the pacemaker 100 may then serve as one electrode.

The implanted medical device comprises an REM (Rapid Eye Movement) sleep detector. REM sleep is a sleep condition characterized by rapidly moving eyes while sleeping. A person in REM sleep typically has an increased heart rate and also typically has an increased variability with regard to the heart rate. Also, the respiration frequency typically increases as well as the respiratory amplitude.

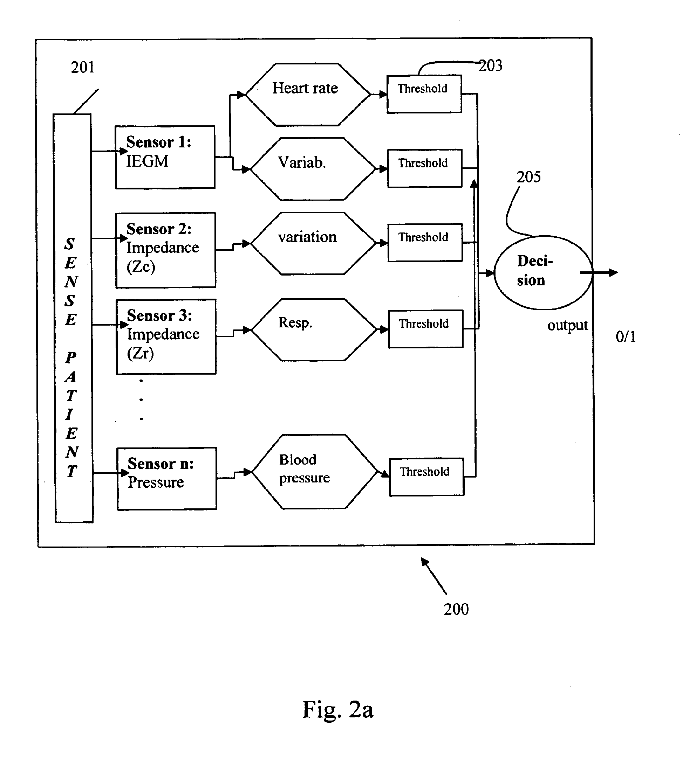

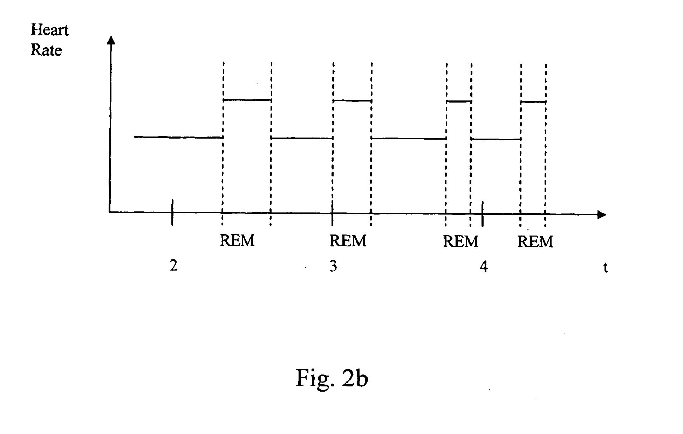

By measuring one or more parameters indicative of REM sleep it is possible to determine if a patient is in REM sleep using an REM sleep detector located in the implantable medical device. In Fig. 2 a diagram of an REM sleep detector 200 adapted to be integrated in an implantable medical device is shown. The REM detector comprises an input terminal 201 for receiving a number of signals sensed by the implantable medical device. In particular the REM detector is adapted to receive sensor signals representing the impedance between two or more electrodes of the implantable medical device as sensed by one or more impedance sensors provided in the implantable medical device. Sensors adapted to generate signals corresponding to the cardiogenic impedance Zc and/ or the respiratory impedance Zr may by employed. In addition the REM detector may receive input sensor signals from sensors adapted to sense signals indicative of, heart rate, heart rate variability, position sensor signals, accelerometer signals, SvO2 (mixed venous oxyhemoglobin) sensor signals and blood pressure sensor signals or any other signals available in the implantable medical device that can serve to facilitate the determination of an existing REM sleep condition.

The sensor signals are used to derive a number of output signals representing different physical parameters such as heart rate, heart rate variability, peak-to-peak variation in heart beat respiration frequency etc, all of which may be extracted from an impedance sensor signal. Each of the measured physical parameters is compared to a threshold value in respective comparison units 203 to determine if an REM sleep condition is present. If all or at least a predetermined number of the measured physiological parameters exceed their respective threshold values the REM sleep detector is adapted to generate an output signal indicating REM sleep in a decision module 205.

In accordance with another embodiment the determination of REM sleep or non REM sleep is made at a later stage and data is only collected and stored during sleep. Afterwards, during data processing, data can be classified as related to REM sleep or Non REM sleep depending on the outcome of an analysis of the collected and stored data. Thus, during sleep data related to physical parameters indicative of REM sleep such as heart rate, heart rate variability, peak-to-peak variation in heart beat respiration frequency etc and which can be captured by the medical device are stored in a memory. Processing of data to determine REM sleep stages is then performed afterwards. In Fig. 2b a schematic view of a heart beat signal used for post capturing detection of REM sleep periods is shown. In Fig. 2b the heart rate during the hours 2 to 4 in the night is schematically shown. Periods with increased heart rate is classified as REM sleep. Other physiological parameters such as heart rate, heart rate variability, peak-to-peak variation in heart beat respiration frequency etc may of course also be used. In particular a number of parameters may be cross correlated to determine periods of REM sleep.

In accordance with the invention, the physiological parameters are sensed using one or more impedance sensors. The impedance sensor signals are then processed to derive signals representing physiological parameters such as heart rate, heart rate variability, peak-to-peak variation in heart beat, respiration frequency etc. Typically there can be provided an impedance sensor for sensing the cardiogenic impedance Zc, i.e. impedance varying with the heart beat, and an impedance sensor for sensing the respiratory impedance Zr, i.e. impedance varying with breathing. Some of the signals that can be derived from the impedance sensors can also be derived from an IEGM sensor if such a sensor is present. For example an IEGM sensor can be used as a supplement to verify the heart beat rate and heart beat variability.

In accordance with another embodiment physiological parameters derived from signals generated by one or many impedance sensors are supplemented by sensor signals generated by other sensors present in the implantable medical device for example sensors adapted to sense signals indicative of, heart rate, heart rate variability, position sensor signals, accelerometer signals, SvO2 (mixed venous oxyhemoglobin) sensor signals and blood pressure sensor signals etc.

Detecting REM sleep can be used to filter out data in a trending algorithm. Trending algorithms are dependent on a data set that is collected under similar conditions in order to generate a reliable trend. A sleeping person is likely to generate data useful and suitable for trending because sampling conditions during sleep are likely to be similar over time. IN addition some heart failure syndromes are known to worsen when a person is lying down.

However, because REM sleep increases the work load on the heart, a trending algorithm should take into account if data collected while a person is sleeping is generated during REM sleep or not.

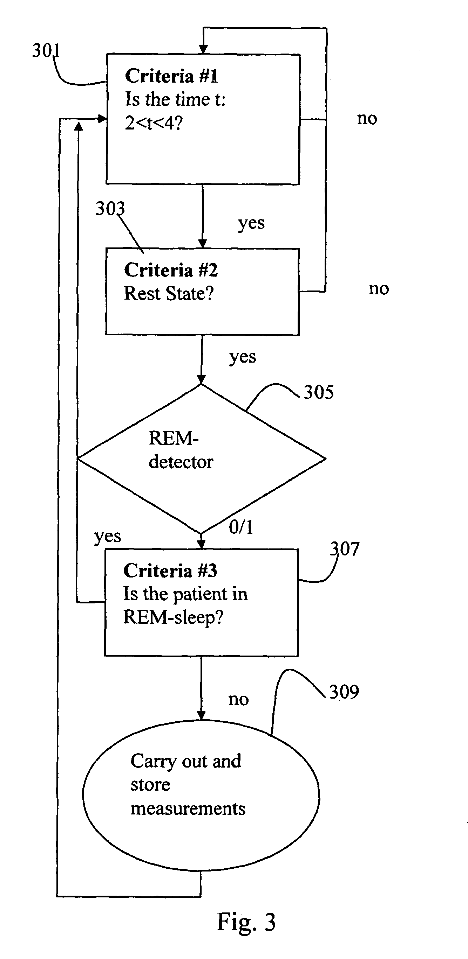

In Fig. 3 a flow chart illustrating steps performed in a procedure when detecting REM sleep and generating trend data is shown. First in a step 301 a first criteria it is checked if the time is such that the patient normally would be asleep. For example the internal clock of the implantable medical device can be checked to see if the time is within some preset time interval, for example between 2 and 4 in the night. Next in a step 303 the procedure checks if the patient is in a rest state. This can be performed by checking readings from an accelerometer or a position sensor if such sensors are present. Next in a step 305 an input signal from an REM sleep detector is received. If the REM sensor output signal indicates that the patient is not in an REM sleep stage in a step 307, trend data is collected for use in a trending algorithm in a step 309.

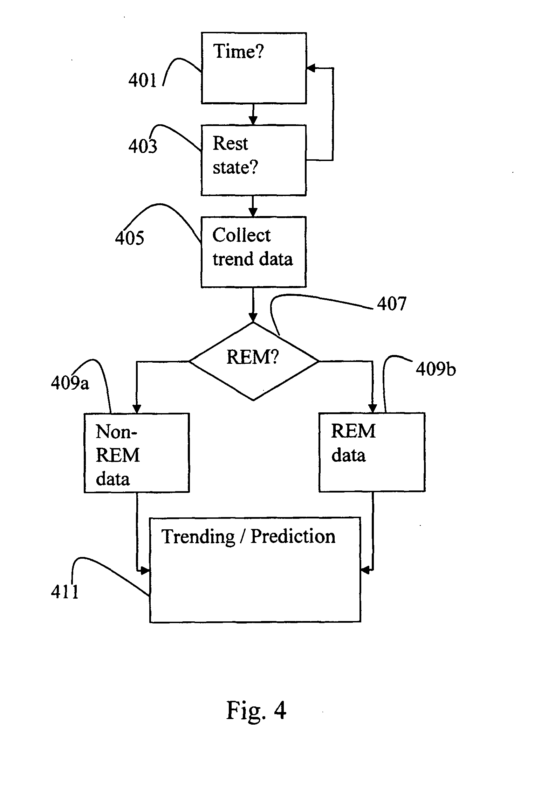

In Fig. 4, a flowchart illustrating different steps performed when collecting trending data in an implantable medical device provided with an REM detector in accordance with another embodiment is shown.

First in a step 401 and 403 it is determined that the patient is sleeping. This can for example be performed as in the embodiment described in conjunction with Fig. 3 by first in the step 401 checking the time of the internal clock of the implantable medical device and then in the step 403 checking that the patient is in a resting state.

If in steps 401 and 403 it is determined that the patient is sleeping, trend data of one or many sensor signals is collected in a step 405. For example, impedance signals such as the cardiogenic Zc and/or respiratory Zr impedance may be collected. Other sensor signals may also be collected depending on the type of diagnosis the trending data is to be used for. Depending on the output from the REM detector the trending data from a sleeping patient is either designated as REM sleep data or sleep data. The determination whether collected data is REM sleep data or sleep data is performed in a step 407.

The data thus collected can then be either stored internally within the implantable medical device or transferred directly to an external device provided to store the trending data. The storing of trending data is performed in a step 409. In either case the trending data collected during REM sleep and regular, non-REM, sleep may be stored in two different data sets 409a and 409 b.

Next, in a step, 411 trending is performed using the trend data collected in REM sleep and/or regular sleep. For example in accordance with one embodiment only data collected during regular sleep is used when performing trending of a physiological parameter. This may be advantageous because the data collected during regular sleep and filtered from REM sleep can be expected to have been collected during very similar conditions thereby increasing the reliability of the trend.

In accordance with another embodiment only data collected during REM sleep is used when performing trending of a physiological parameter. This may be advantageous because the data collected during REM sleep, apart from being collected during similar conditions, is also collected during a higher cardiogenic workload, which in turn increases the likelihood of detecting an abnormal state early since abnormal states usually are first spotted during a higher workload.

In accordance with yet another embodiment both REM sleep data and regular sleep data are combined in a suitable way to create a trend for a combined set of data. For example, the ratio between the REM sleep data and the regular sleep data from time periods close in time can be trended against the corresponding ratio at other times. Hereby the ratio is trended and that may provide an even more precise trend of the trended parameter. Other relationships between data collected during REM sleep and non-REM sleep may also be studied and trended. For example, the difference or a correlation may be studied.

Stored data can also be further processed. For example during a period of sleep a number of REM sleep sessions and regular sleep sessions may occur. For each such session only a number of parameters representing the data to be trended may be permanently stored and used by the trending tool used. Thus, if trending data is only collected from the implantable medical device at some periodic interval, such as bi-weekly, the implantable medical device can have two memories, one first memory for storing recently collected data and another second memory for storing parameters representing the trended parameter. Hereby the first memory can be emptied as soon as the relevant data has been processed and stored as one or many data values in the second memory. The trending tool then only has to upload data from the second memory to generate the trend for the trended parameter(s) which saves time and memory.

Once collected the data used for trending can be transferred to a device located outside the implantable medical device. As stated above this can be performed either in real time or, if the implantable medical device is provided with a memory at a time interval such that the memory does not become full before being transferred to the outside device.

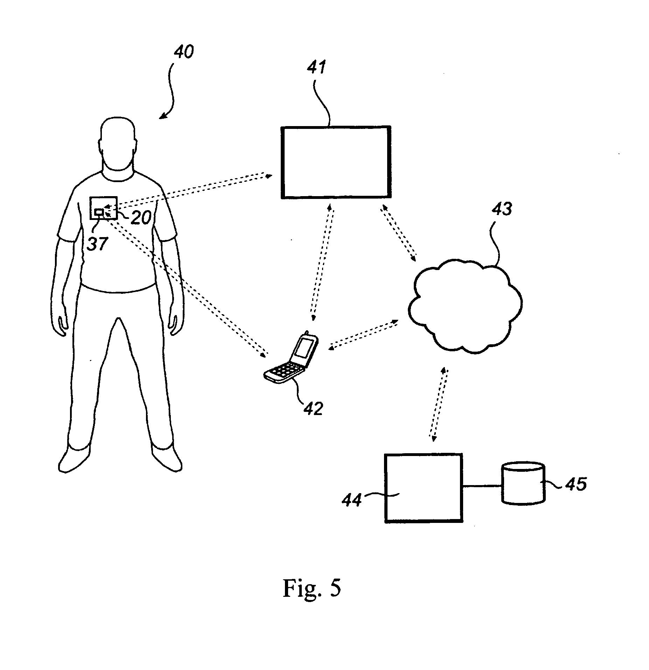

In Fig. 5, transfer of information to/from an implantable medical device is schematically shown. Thus, an implantable medical device 20 implanted in a patient 40 comprises a device 37 for transmitting information from the device 20 to entities outside the patient. The device 37 may also act to receive data or instructions from outside entities. For example, the implantable medical device can communicate with a cellular device such as a mobile telephone 42 or a stationary transmitter/receiver 41. The cellular device 42 or the stationary transmitter/receiver 41 can then be connected to a processor unit 44 for example via a network 43. The processor unit can for example be a trending tool adapted to receive trend data to be processed by the trending tool 44. The processed data can then be displayed to a doctor treating the patient or stored in a memory 45 for future use.

Using the device as described herein will provide an efficient REM sleep detector using an impedance signal as input signal, and which hence can detect REM sleep using only sensor signals present in an implantable medical device and which hence enables REM sleep detection of a patient carrying an implantable medical device such as an implanted pacemaker or a cardioverter/defibrillator. The methods and devices as described herein may also be used in an implantable medical monitor device used for monitoring physiological parameters.

The provision of a REM sleep detector, in particular inside an implantable medical device, enables better trending and prediction of monitored conditions or diseases. This is because

REM sleep data can be separated from other data whereby stable and uniform conditions for data collection are achieved. Stable and uniform data sampling conditions is one of the most important if not the most important aspect for obtaining a reliable result when using data in a trending tool. This is because no matter how a sophisticated trending/prediction tool that is used, if data is not comparable or representative the tool cannot provide a useful output.

## Claims

1. An implantable medical device (20,100) comprising a unit for collecting trend data to be used for trending at least one physiological parameter of a person, said device comprises:
means (200) for detecting if said person is in REM sleep, and
means (200) for storing data related to non-REM sleep as a first set of data,
an internal clock, and
means for determining that the person is asleep based on whether a time from said internal clock is within a preset time interval and readings from an accelerometer or a position sensor indicates a rest state of said person, wherein
said means (200) for detecting REM sleep is adapted to receive sensor signals representing a cardiogenic impedance between two or more electrodes of said implantable medical device as sensed by one or more impedance sensors,
said means (200) for detecting REM sleep is adapted to extract a heart rate and/or a heart rate variability from said sensor signals representing said cardiogenic impedance, and
said means (200) for detecting REM sleep comprises a respective comparison unit (203) **characterized in that** said comparison unit is adapted to compare said heart rate and/or said heart rate variability to a respective threshold value to determine if said person is in said REM sleep.

2. The device according to claim 1, further comprising:
means for storing data related to REM sleep as a second set of data.

3. The device according to claim 1 or 2, further comprising:
means for performing trending and/or prediction of said at least one physiological parameter using only data collected during non-REM sleep.

4. The device according to claim 2, further comprising:
means for performing trending and/or prediction of said at least one physiological parameter using only data collected during REM sleep.

5. The device according to claim 2, further comprising:
means for performing trending and/or prediction of said at least one physiological parameter using a combination of data collected during non-REM sleep and data collected during REM sleep.

6. The device according to claim 5, further comprising:
means for forming a ratio between non-REM sleep data and REM sleep data to be used as trend/prediction parameter.

7. The device according to claim 6, further comprising:
means for forming the ratio from samples collected from consecutive REM/non-REM sleep periods.

## Patentansprüche

1. Implantierbares Medizinprodukt (20, 100), umfassend eine Einheit zum Sammeln von Trenddaten, die zur Trendermittlung von mindestens einem physiologischen Parameter einer Person verwendet werden sollen, wobei das Produkt Folgendes umfasst:
ein Mittel (200) zum Erkennen, ob sich die Person im REM-Schlaf befindet, und
ein Mittel (200) zum Speichern von den Nicht-REM-Schlaf betreffenden Daten als ersten Datensatz,
eine integrierte Uhr, und
Mittel zum Feststellen, dass die Person schläft, anhand dessen, ob eine Zeit der integrierten Uhr innerhalb eines vorgegebenen Zeitintervalls liegt und Anzeigewerte eines Beschleunigungsmessers oder eines Lagesensors einen Ruhezustand der Person anzeigen, wobei
das Mittel (200) zum Erkennen von REM-Schlaf so angepasst ist, dass es Sensorsignale empfängt, die eine kardiogene Impedanz zwischen zwei oder mehr Elektroden des implantierbaren Medizinprodukts darstellen, die von einem oder mehreren Impedanzsensoren erfasst wird,
das Mittel (200) zum Erkennen von REM-Schlaf so angepasst ist, dass es eine Herzfrequenz und/oder eine Herzfrequenzvariabilität aus den Sensorsignalen, die die kardiogene Impedanz darstellen, entnimmt, und
das Mittel (200) zum Erkennen von REM-Schlaf eine entsprechende Vergleichseinheit (203) umfasst, **dadurch gekennzeichnet, dass** die Vergleichseinheit so angepasst ist, dass sie die Herzfrequenz und/oder die Herzfrequenzvariabilität mit einem jeweiligen Schwellenwert vergleicht, um festzustellen, ob sich die Person im REM-Schlaf befindet.

2. Produkt nach Anspruch 1, ferner umfassend:
Mittel zum Speichern von den REM-Schlaf betreffenden Daten als zweiten Datensatz.

3. Produkt nach Anspruch 1 oder 2, ferner umfassend:
Mittel zum Durchführen einer Trendermittlung und/ oder Vorhersage des mindestens einen physiologischen Parameters nur unter Verwendung von Daten, die während des Nicht-REM-Schlafs gesammelt werden.

4. Produkt nach Anspruch 2, ferner umfassend:
Mittel zum Durchführen einer Trendermittlung und/oder Vorhersage des mindestens einen physiologischen Parameters nur unter Verwendung von Daten, die während des REM-Schlafs gesammelt werden.

5. Produkt nach Anspruch 2, ferner umfassend:
Mittel zum Durchführen einer Trendermittlung und/oder Vorhersage des mindestens einen physiologischen Parameters unter Verwendung einer Kombination aus Daten, die während des Nicht-REM-Schlafs gesammelt werden, und Daten, die während des REM-Schlafs gesammelt werden.

6. Produkt nach Anspruch 5, ferner umfassend:
Mittel zum Bilden eines Verhältnisses zwischen Nicht-REM-Schlaf-Daten und REM-Schlaf-Daten, das als Trend-/Vorhersageparameter verwendet werden soll.

7. Produkt nach Anspruch 6, ferner umfassend:
Mittel zum Bilden des Verhältnisses aus Abtastwerten, die aus aufeinanderfolgenden REM-/Nicht-REM-Schlafzeiträumen erhoben werden.

## Revendications

1. Dispositif médical implantable (20, 100) comprenant une unité pour collecter des données de tendance destinées à être utilisées pour déterminer une tendance d'au moins un paramètre physiologique d'une personne, ledit dispositif comprenant :
un moyen (200) pour détecter si ladite personne est dans un sommeil paradoxal, et
un moyen (200) pour enregistrer des données relatives au sommeil non paradoxal sous forme d'un premier jeu de données,
une horloge interne, et
des moyens pour déterminer que la personne est endormie selon qu'un temps de ladite horloge interne est dans un intervalle de temps préréglé et que des valeurs indiquées d'un accéléromètre ou d'un capteur de position indiquent un état de repos de ladite personne ou pas, dans lequel
ledit moyen (200) pour détecter un sommeil paradoxal est adapté pour recevoir des signaux de capteur représentant une impédance cardiogénique entre deux ou plus d'électrodes dudit dispositif médical implantable telle que détectée par un ou plusieurs capteurs d'impédance,
ledit moyen (200) pour détecter un sommeil paradoxal est adapté pour extraire une fréquence cardiaque et/ou une variabilité de fréquence cardiaque desdits signaux de capteur représentant ladite impédance cardiogénique, et
ledit moyen (200) pour détecter un sommeil paradoxal comprend une unité de comparaison (203) respective, **caractérisé en ce que** ladite unité de comparaison est adaptée pour comparer ladite fréquence cardiaque et/ou ladite variabilité de fréquence cardiaque à une valeur de seuil respective pour déterminer si ladite personne est dans ledit sommeil paradoxal.

2. Dispositif selon la revendication 1, comprenant en outre :
des moyens pour enregistrer des données relatives au sommeil paradoxal sous forme d'un second jeu de données.

3. Dispositif selon la revendication 1 ou 2, comprenant en outre :
des moyens pour effectuer la détermination d'une tendance et/ou prédiction dudit au moins un paramètre physiologique en utilisant uniquement des données collectées pendant le sommeil non paradoxal.

4. Dispositif selon la revendication 2, comprenant en outre :
des moyens pour effectuer la détermination d'une tendance et/ou prédiction dudit au moins un paramètre physiologique en utilisant uniquement des données collectées pendant le sommeil paradoxal.

5. Dispositif selon la revendication 2, comprenant en outre :
des moyens pour effectuer la détermination d'une tendance et/ou prédiction dudit au moins un paramètre physiologique en utilisant une combinaison de données collectées pendant le sommeil non paradoxal et de données collectées pendant le sommeil paradoxal.

6. Dispositif selon la revendication 5, comprenant en outre :
des moyens pour former un rapport entre des données relatives au sommeil non paradoxal et des données relatives au sommeil paradoxal destiné à être utilisé en tant que paramètre de tendance/prédiction.

7. Dispositif selon la revendication 6, comprenant en outre :
des moyens pour former le rapport à partir d'échantillons collectés dans des périodes consécutives de sommeil paradoxal/non paradoxal.
